# EUROPEAN PATENT APPLICATION

(11) **EP 3 260 048 A1**
(43) Date of publication of application: **27.12.2017**
(21) Application number: 16752342.2
(22) Date of filing: 09.02.2016
(51) Int. Cl.: A61B 8/14, G06T 1/00

(54) **ULTRASOUND DIAGNOSTIC DEVICE**

(30) Priority: 20.02.2015 JP 2015031587
(71) Applicant: Hitachi, Ltd., Tokyo 100-8280 (JP)
(72) Inventor: KOBAYASHI, Masaki, Mitaka-shi Tokyo 181-8622 (JP); INOUE, Nobuyasu, Mitaka-shi Tokyo 181-8622 (JP); MURASHITA, Masaru, Mitaka-shi Tokyo 181-8622 (JP); NAGASE, Yuko, Mitaka-shi Tokyo 181-8622 (JP); MAEDA, Toshinori, Mitaka-shi Tokyo 181-8622 (JP); MITANI, Yuki, Mitaka-shi Tokyo 181-8622 (JP)
(74) Representative: Strehl Schübel-Hopf & Partner
(86) International application number: PCT/JP2016/053736
(87) International publication number: WO 2016/132956

(57) **Abstract**

On the basis of voxel data for a plurality of voxels constituting a set of ultrasound volume data, a voxel group identifying unit 50 identifies, in said ultrasound volume data, one or more voxel groups formed by a plurality of voxels in which voxel data satisfy a condition of being linked. On the basis of the voxel data for a plurality of voxels corresponding to each voxel group to be displayed, from among the one or more identified voxel groups, an image forming unit 80 forms an ultrasound image in which the voxel groups to be displayed are indicated clearly in a selective manner. It is thus possible for a three-dimensional image to be formed in such a way that one part of the image, such as floating matter in the amniotic fluid, does not interfere with another part of the image, such as a fetus.

## Description

### TECHNICAL FIELD

The present invention relates to an ultrasound diagnostic device.

### BACKGROUND

An ultrasound diagnostic device is used for diagnosis of tissues and the like in a living body and is particularly useful in diagnosis of a fetus. Conventionally, there have been proposed various techniques related to diagnosis of a fetus (refer to Patent Documents 1 to 3). For example, based on an ultrasound image showing the figure of a fetus in the womb, the development of the fetus and the like can be diagnosed.

### CITATION LIST

### PATENT LITERATURE

Patent Document 1: JP 2007-252725 A
Patent Document 2: JP 2006-223712 A
Patent Document 3: JP 2011-98191 A
Patent Document 4: JP 2011-83439 A

### SUMMARY

### TECHNICAL PROBLEM

In addition to the fetus, the placenta and floating matters in the amniotic fluid are also present in the womb, and upon displaying the figure of the fetus such as, for example, the face of the fetus, in an ultrasound image, the placenta, the floating matters, and the like may become obstacles for display. Therefore, it is desirable to enable clear display, in a selective manner, of an image portion of the fetus in an ultrasound image showing the fetus, while removing image portions of the placenta and the floating matters that become obstacles for display. However, in many cases, floating matters particularly float in the form of a plurality of discrete masses in the amniotic fluid, and it is not easy to remove the plurality of masses.

The present invention has been conceived in view of the background described above, and an object thereof is to provide a technique of clearly displaying a display target such as, for example, a fetus, in a selective manner in an ultrasound image.

### SOLUTION TO PROBLEM

An ultrasound diagnostic device which is preferable as a specific example of the present invention is characterized in that the device has an identification unit that identifies, in ultrasonic volume data, one or more voxel groups composed of a plurality of voxels having voxel data that satisfy connection conditions, based on voxel data of a plurality of voxels constituting the volume data, and an image forming unit that forms, based on voxel data of a plurality of voxels corresponding to a voxel group that is a display target among the identified one or more voxel groups, an ultrasound image clearly displaying the display target in a selective manner.

The volume data that are an object to be processed by the above device are formed based on echo data collected stereoscopically (in three dimensions) from a three-dimensional region by, for example, transmitting and receiving ultrasonic waves. The volume data are composed of, for example, voxel data of a plurality of voxels arranged in three dimensions (provided with addresses in three dimensions). Voxel data of each voxel can be obtained based on the echo data. As a matter of course, the echo data may be used as the voxel data without change.

The identification unit of the above device then identifies one or more voxel groups in the volume data. One voxel group corresponds to, for example, one tissue or one tissue piece. For example, a mass composed of a plurality of adjacent voxels that can be regarded to have comparable luminance values (values based on the voxel data), is recognized as one voxel group. Specifically, for example, in the case of volume data in the womb, a plurality of voxels corresponding to the face of the fetus constitute one voxel group, and each of a plurality of floating matters is identified as one voxel group.

Furthermore, at least one group of one or more voxel groups identified by the identification unit of the above device is set as a display target. For example, when only one voxel group is identified, that voxel group is set as a display target, and when a plurality of voxel groups are identified, for example, one or more voxel groups specified by the user are set as display targets. In addition, it is also possible, for example, to specify voxel groups that become non-display targets by the user, and regard voxel groups other than them as display targets. For example, when volume data in the womb constitute a target to be processed, a voxel group corresponding to the face of the fetus is set as a display target, and voxel groups corresponding to the floating matters in the amniotic fluid are set as non-display targets.

The image forming unit of the above device forms an ultrasound image that clearly displays the display target in a selective manner. Clear display of the display target in a selective manner includes display of an image portion of that display target with higher priority than other image portions. For example, it is preferable that the image portion of the display target is displayed without being disturbed by other image portions. Specifically, only the image portion of the display target may be displayed, or the image portion of the display target may be displayed on the frontmost side (viewpoint side of an image). The display target may also be displayed clearly by differentiating display modes between the display target and the non-display targets by, for example, displaying as transparent the image portions other than the display target.

With this process, it becomes possible to clearly display a desired display target in a selective manner in an ultrasound image. For example, it becomes possible to clearly display the face of a fetus in an ultrasound image showing the inside of the womb, without being disturbed by floating matters in the amniotic fluid.

In a preferable specific example, the ultrasound diagnostic device is characterized in that it further has a determination unit that determines, for each voxel group identified by the identification unit, whether that voxel group is a display target, and a storage unit that stores reference data indicating, for each voxel group, whether that voxel group is a display target.

In the preferable specific example, the ultrasound diagnostic device is characterized in that the storage unit stores, as the reference data, reference volume data composed of mark data indicating, for each voxel of the plurality of voxels constituting the volume data, whether that voxel is a display target, and in that the determination unit updates the reference volume data such that the mark data of a plurality of voxels belonging to a voxel group that is determined to be the display target show the display target.

In the preferable specific example, the ultrasound diagnostic device further has a setting unit that sets a reference point within the volume data based on instructions from the user, and the ultrasound diagnostic device is characterized in that the identification unit identifies the voxel group composed of the plurality of voxels that satisfy the connection conditions, using a voxel corresponding to the reference point as an origin.

In the preferable specific example, the ultrasound diagnostic device is characterized in that the setting unit sets, as the reference point, a specified point which is set by the user in a tomographic image corresponding to a cross section in the volume data.

In the preferable specific example, the ultrasound diagnostic device is characterized in that a three-dimensional image showing the inside of the volume data stereoscopically is formed based on pixel data obtained from a plurality of rays passing through the inside of the volume data, and that the setting unit sets the reference point on a ray corresponding to pixel data of a specified point set by the user in the three-dimensional image.

In the preferable specific example, the ultrasound diagnostic device is characterized in that the setting unit searches a voxel region corresponding to the face of a fetus based on voxel data of a plurality of voxels constituting the ray corresponding to the specified point, and sets the reference point within the voxel region.

In the preferable specific example, the ultrasound diagnostic device is characterized in that the identification unit identifies, as the voxel group, a plurality of voxels that satisfy the connection conditions using a voxel corresponding to the reference point as an origin and that are within a specified distance from the reference point.

In the preferable specific example, the ultrasound diagnostic device is characterized in that the identification unit identifies, as the voxel group, a plurality of voxels that satisfy the connection conditions using a voxel corresponding to the reference point as an origin and that are less than a specified number of voxels.

In the preferable specific example, the ultrasound diagnostic device is characterized in that one or more voxel groups identified by the identification unit include a voxel group corresponding to the face of the fetus which is a display target, and in that the image forming unit forms an ultrasound image clearly displaying the face of the fetus in a selective manner based on voxel data of a plurality of voxels constituting the voxel group corresponding to the face of the fetus.

In the preferable specific example, the ultrasound diagnostic device is characterized in that, based on the voxel data of the plurality of voxels corresponding to the face of the fetus and voxel data of a plurality of voxels that satisfy conditions for a background tissue in the volume data, the image forming unit forms an ultrasound image that shows the face of the fetus and the background tissue.

### ADVANTAGEOUS EFFECTS OF INVENTION

With the present invention, it becomes possible to clearly display a desired image portion in an ultrasound image in a selective manner. For example, it becomes possible to clearly display the face of a fetus in an ultrasound image showing the inside of the womb, without being disturbed by floating matters in the amniotic fluid.

### BRIEF DESCRIPTION OF DRAWINGS

[FIG. 1] FIG. 1 is an overall block diagram of a preferable ultrasound diagnostic device in implementation of the present invention.
[FIG. 2] FIG. 2 is a diagram showing a specific example of volume data.
[FIG. 3] FIG. 3 is a diagram showing specific examples of display images.
[FIG. 4] FIG. 4 is a diagram showing a specific example in which a reference point is set from a specified point in a three-dimensional image.
[FIG. 5] FIG. 5 is a diagram for illustrating the principle of a region growing method.
[FIG. 6] FIG. 6 is a diagram showing a specific example of a plurality of voxel groups.
[FIG. 7] FIG. 7 is a diagram showing a specific example of reference data.
[FIG. 8] FIG. 8 is a diagram showing specific examples of three-dimensional images.
[FIG. 9] FIG. 9 is a diagram showing a specification example 1 of a display target and a non-display target.
[FIG. 10] FIG. 10 is a diagram showing a specification example 2 of display targets and a non-display target.
[FIG. 11] FIG. 11 is a diagram showing a specific example of processing performed by the ultrasound diagnostic device in FIG. 1.
[FIG. 12] FIG. 12 is a diagram showing an image processing example in which a background is added to a display target.

### DESCRIPTION OF EMBODIMENTS

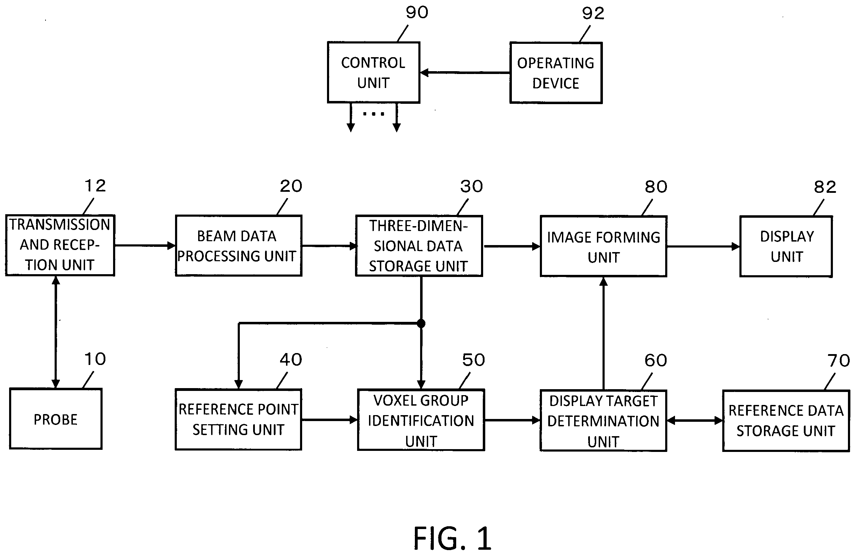

FIG. 1 is an overall block diagram of a preferable ultrasound diagnostic device in implementation of the present invention. A probe 10 is an ultrasonic probe that transmits and receives ultrasonic waves to/from a three-dimensional space including an object to be diagnosed. The probe 10 has a plurality of transducer elements, and each transducer element transmits ultrasonic waves to the three-dimensional space in response to a transmission signal obtained from a transmission and reception unit 12. Each transducer element that receives a reflected ultrasonic wave (echo) from the three-dimensional space also outputs a received wave signal to the transmission and reception unit 12 according to that reflected wave.

The transmission and reception unit 12 outputs a transmission signal corresponding to each of the plurality of transducer elements of the probe 10 to execute transmission control of the probe 10. By that transmission control, an ultrasonic transmission beam is formed, and the transmission beam is scanned in the three-dimensional space.

A beam data processing unit 20 obtains from the transmission and reception unit 12a plurality of received wave signals corresponding to the plurality of transducer elements of the probe 10, and performs beam forming processing, such as phasing addition processing, on the plurality of received wave signals. By this processing, ultrasonic reception beams are formed and scanned in the three-dimensional space. That is, the reception beams are scanned in the three-dimensional space while their beam addresses are differentiated, and the beam data processing unit 20 forms a plurality of pieces of line data corresponding to the plurality of beam addresses. Each piece of line data is composed of a plurality of pieces of echo data.

In this way, the ultrasonic beams (transmission beams and their corresponding reception beams) are scanned stereoscopically in the three-dimensional space, and the plurality of pieces of echo data can be obtained from the inside of the three-dimensional space. The probe 10 is a 3D probe that scans the ultrasonic beams in the three-dimensional space and collects the pieces of echo data stereoscopically. For example, by mechanically moving a scanning plane that is electronically formed by a plurality of transducer elements arranged in one dimension (1D array transducers), ultrasonic beams are scanned in three dimensions. A plurality of transducer elements arranged in two dimensions (2D array transducers) may also be controlled electronically to scan ultrasonic beams in three dimensions.

A three-dimensional data storage unit 30 stores volume data based on the plurality of pieces of echo data obtained from the inside of the three-dimensional space. For example, the beam data processing unit 20 performs coordinate conversion processing or the like on the plurality of pieces of echo data and converts the data into a preferable coordinate system in a later process. The three-dimensional data storage unit 30 then stores the data after conversion as volume data.

For example, the beam data processing unit 20 performs three-dimensional coordinate conversion processing and interpolation processing or the like on the plurality of pieces of echo data obtained in the ultrasonic scanning coordinate system (e.g. r, θ, ϕ coordinate system), thereby forming volume data corresponding to a three-dimensional rectangular coordinate system (x-y-z coordinate system), and the three-dimensional data storage unit 30 stores the volume data.

The beam data processing unit 20 may perform two-dimensional coordinate conversion processing on the plurality of pieces of echo data, thereby converting, of the scanning coordinate system, the r, θ coordinate system corresponding to the scanning plane to the rectangular coordinate system (x-y coordinate system), and the three-dimensional data storage unit 30 may store the data after conversion as volume data. In addition, the plurality of pieces of echo data, which are obtained by scanning ultrasonic waves stereoscopically in the three-dimensional space, may be provided with addresses corresponding to the scanning coordinate system corresponding to stereoscopic scanning of ultrasonic waves (e.g. r, θ, ϕ coordinate system), and stored in three-dimensional data storage unit 30 as volume data.

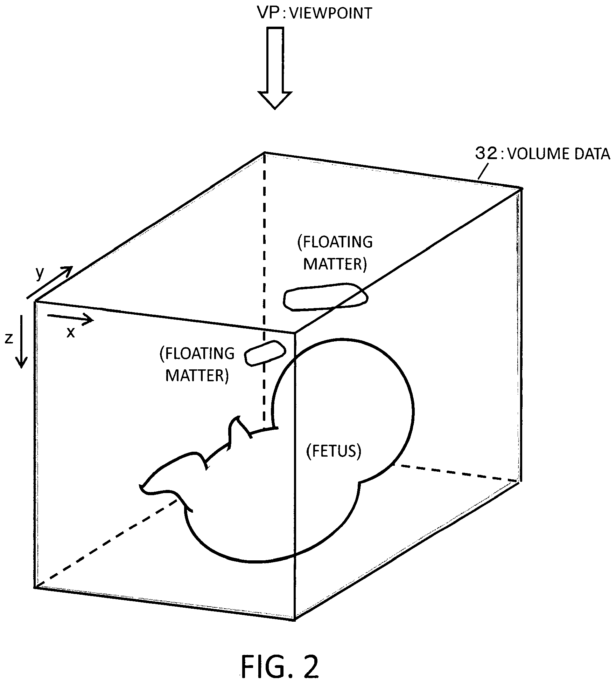

FIG. 2 is a diagram showing a specific example of volume data 32. FIG. 2 shows the volume data 32 in the xyz rectangular coordinate system corresponding to the three-dimensional space including a fetus which is a preferable specific example of an object to be diagnosed. The volume data 32 are composed of voxel data of a plurality of voxels provided with addresses in three dimensions in the xyz rectangular coordinate system.

As shown in the specific example in FIG. 2, because not only the fetus but also floating matters in the amniotic fluid are present in the womb, portions corresponding to the fetus and the floating matters in the amniotic fluid are included in the volume data 32 including the fetus in the three-dimensional space. The placenta and a portion of the uterine wall can be included in the volume data 32.

Referring to FIG. 1 again, an image forming unit 80 forms an ultrasound image based on the volume data 32 (FIG. 2) stored in the three-dimensional data storage unit 30. Based on the volume data 32 corresponding to the three-dimensional space including the object to be diagnosed, the image forming unit 80 forms a three-dimensional ultrasound image stereoscopically showing that object to be diagnosed. A preferable specific example of a three-dimensional ultrasound image is a rendering image that can be obtained by known volume rendering processing.

In volume rendering processing, for example, as shown in the specific example in FIG. 2, a virtual viewpoint VP is set on the outside of the volume data 32 corresponding to the three-dimensional space, and a screen is virtually set as a two-dimensional plane on the opposite side to the viewpoint VP with the volume data 32 therebetween. A plurality of rays (perspective rays) are defined based on that viewpoint VP as a reference. Each ray is set so as to penetrate through the volume data 32. With this setting, on each ray, or near each ray, pieces of voxel data of a plurality of voxels corresponding to that ray are associated. When voxel calculation is then performed, per ray, on a plurality of voxels corresponding to that ray from the viewpoint VP side in a sequential manner based on the rendering method, a pixel value corresponding to that ray is determined as a result of final voxel calculation. By mapping on the screen a plurality of pixel values obtained from the plurality of rays, a rendering image can be obtained.

Further, the image forming unit 80 in FIG. 1 forms a tomographic image corresponding to a cross section in the volume data 32. The image forming unit 80 forms, for example, tomographic images corresponding to three cross sections that are orthogonal to each other (three orthogonal cross sections) in the volume data 32. The ultrasound images formed in the image forming unit 80 are displayed on a display unit 82 as display images.

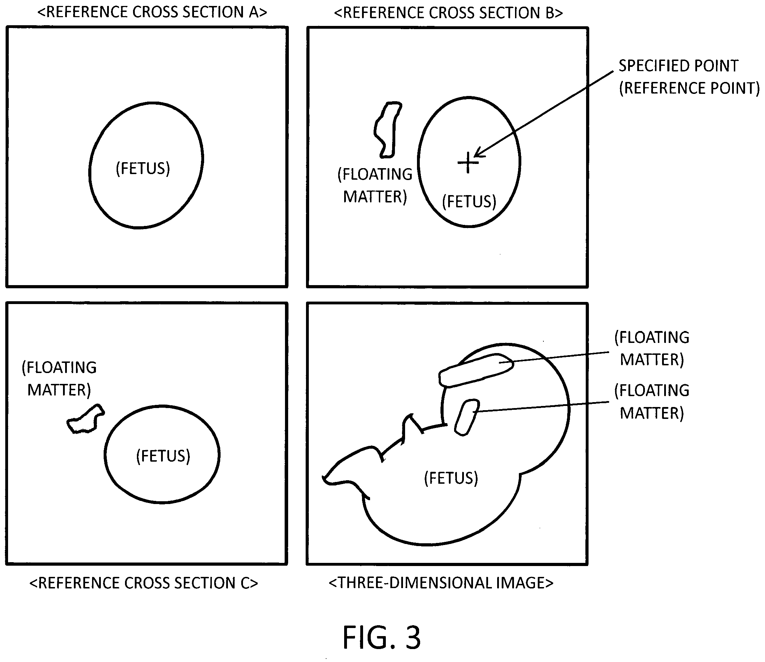

FIG. 3 is a diagram showing specific examples of display images. A three-dimensional image in FIG. 3 is a specific example of a rendering image formed by the image forming unit 80 and is obtained based on the volume data 32 in FIG. 2. In the specific examples shown in FIG. 3, the fetus, which is a preferable specific example of the object to be diagnosed, is shown stereoscopically in the three-dimensional image.

A reference cross section A, a reference cross section B, and a reference cross section C shown in FIG. 3 are specific examples of three orthogonal cross sections formed by the image forming unit 80 and are obtained based on the volume data 32 shown in FIG. 2. For example, the three orthogonal cross sections are set so as to intersect with each other at the center of the volume data 32 shown in FIG. 2. It is also possible to set the three orthogonal cross sections so as to intersect with each other at a point other than the center of the volume data 32. Cross sections are not limited to the three orthogonal cross sections, and it is alternatively possible to set any cross section in the volume data 32 and form a tomographic image corresponding to that cross section. As a matter of course, positions and inclinations of the cross sections in the volume data 32 may be made adjustable as appropriate.

Now, there are also the placenta and floating matters in the amniotic fluid in the womb, and upon showing the figure of the fetus, such as the face of the fetus, in an ultrasound image, the placenta, the floating matters, and the like may become obstacles for display. For example, if a three-dimensional image (rendering image) of the fetus viewed from the viewpoint VP is formed based on the volume data 32 shown in FIG. 2, as shown in the three-dimensional image in FIG. 3, the floating matters that are nearer to the viewpoint VP than the fetus are displayed on the front side (viewpoint VP side) of the fetus, and this may become an obstacle for display of the fetus.

Therefore, the ultrasound diagnostic device in FIG. 1 has a function of clearly displaying an image portion of a display target in a selective manner such that, for example, the image portion of the display target (the fetus or the like) is not disturbed by other image portions (the floating matter or the like). That function will be described below. In the following description, the components shown in FIG. 1 (units with the reference numbers) will be assigned the reference numbers used in FIG. 1.

A reference point setting unit 40 sets a reference point in the volume data 32 (FIG. 2) based on instructions from the user. The reference point setting unit 40 sets, as the reference point, a specified point that is, for example, set by the user in a tomographic image corresponding to a cross section in the volume data 32.

FIG. 3 shows a specific example of a specified point set by the user in a tomographic image corresponding to a cross section in the volume data 32 (FIG. 2). The user operates an operating device 92, such as a track ball, while looking at, for example, the ultrasound image of FIG. 3 displayed on the display unit 82, and determines a position of a specified point, thereby setting the specified point in the tomographic image. With this operation, the specified point is set at an image portion which is a display target.

FIG. 3 shows a specific example where a specified point is set at an image portion of the fetus in the reference cross section B. The reference cross section B is a cross section set in the volume data 32, and its geometric position (including its inclination) in the xyz rectangular coordinate system is known. Therefore, once one specified point is set in the reference cross section B, the coordinates of that specified point in the xyz rectangular coordinate system are determined. Accordingly, the reference point setting unit 40 sets a reference point at the position (coordinates) of the specified point, which is set in the reference cross section B, in the xyz rectangular coordinate system.

The user may also set a specified point in a three-dimensional image. When the specified point is set in the three-dimensional image, the reference point setting unit 40 sets a reference point on a ray corresponding to a pixel of the reference point set by the user.

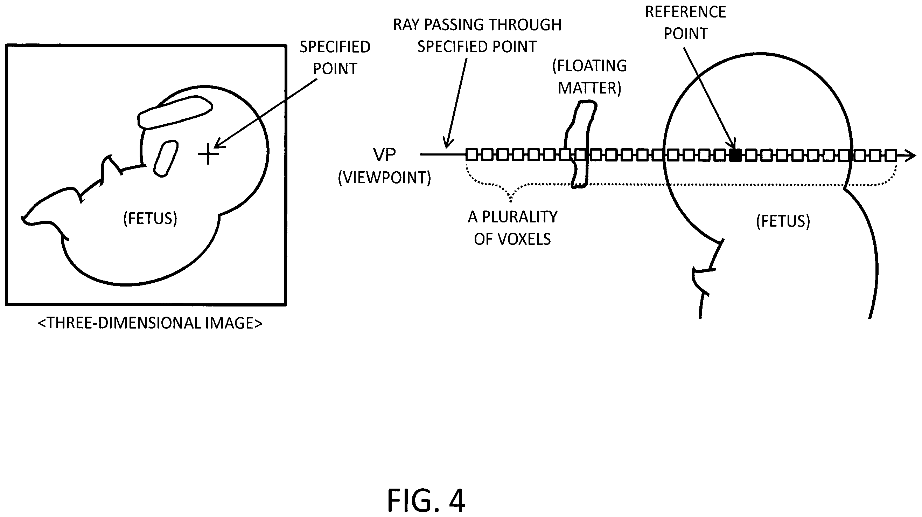

FIG. 4 is a diagram showing a specific example in which a reference point is set from a specified point in a three-dimensional image. For example, the user operates the operating device 92 such as a track ball, while looking at, for example, a three-dimensional image displayed on the display unit 82, and determines a position of a specified point to thereby set the specified point in the three-dimensional image. With this operation, the specified point is set at an image portion which is a display target.

FIG. 4 shows a specific example where the specified point is set at an image portion of the fetus in the three-dimensional image. The three-dimensional image is, for example, a rendering image obtained by known volume rendering processing. As already described, the rendering image can be obtained by mapping on the screen a plurality of pixel values obtained from a plurality of rays. Therefore, once the specified point is set in the rendering image, a pixel at a position corresponding to that specified point can be determined, and one ray corresponding to that pixel can be further identified.

Accordingly, the reference point setting unit 40 identifies one ray corresponding to the specified point, searches a voxel region corresponding to the fetus based on pieces of voxel data of a plurality of voxels corresponding to that ray, and sets a reference point within that voxel region.

Specifically, as shown in FIG. 4, concerning a plurality of voxels arranged along a ray corresponding to the specified point (passing through the specified point), pieces of voxel data of the voxels are sequentially confirmed from the one on the viewpoint VP side. Voxel data of each voxel has a value (voxel value) according to the size of echo data at a position of that voxel. Therefore, in the amniotic fluid where the fetus is included, the voxels corresponding to the amniotic fluid have relatively small voxel values, while the voxels corresponding to the fetus have relatively large voxel values. The size of the voxel value can be distinguished by binarization processing, for example.

The reference point setting unit 40 compares the voxel values of the voxels with a threshold, and determines that the voxels having voxel values greater than the threshold (equal to or greater than the threshold) correspond to the fetus, and that the voxels having voxel values equal to or less than the threshold (less than the threshold) correspond to the amniotic fluid. However, because floating matters in the amniotic fluid also have voxel values larger than that of the amniotic fluid, when, for example, as in the specific example shown in FIG. 4, there is a floating matter on the ray passing through the specified point, a voxel corresponding to the floating matter may be misunderstood as a voxel corresponding to the fetus in determination by binarization processing alone.

Therefore, the reference point setting unit 40 determines that, if voxels having voxel values equal to or greater than (greater than) the threshold continue a reference number of times or more (e.g. any one of five to ten), a region including the plurality of consecutive voxels is a voxel region corresponding to the fetus, and sets a reference point of the fetus in that voxel region. That is, because the floating matter is smaller than the fetus, it is determined that the plurality of consecutive voxels are a voxel region corresponding to the fetus only when voxels having values equal to or greater than the threshold continue the reference number of times or more.

The reference point setting unit 40 then sets a reference point within the voxel region corresponding to the fetus. The reference point is set at the center of the voxel region, for example. The reference point may, of course, be set at a position other than the center of the voxel region corresponding to the fetus.

In addition, if the user sets a specified point in an object other than the fetus, the reference point setting unit 40 sets a reference point of the object corresponding to that specified point. For example, when the user sets specified points for a plurality of objects, the reference point setting unit 40 sets, for each object, a reference point of that object.

Once the reference point setting unit 40 sets the reference point, a voxel group identification unit 50 identifies one or more voxel groups composed of a plurality of voxels having pieces of voxel data that satisfy connection conditions in the volume data 32 (FIG. 2) in which the reference point is set. Upon identifying one or more voxel groups, any known techniques may be used, and a typical example is a region growing method.

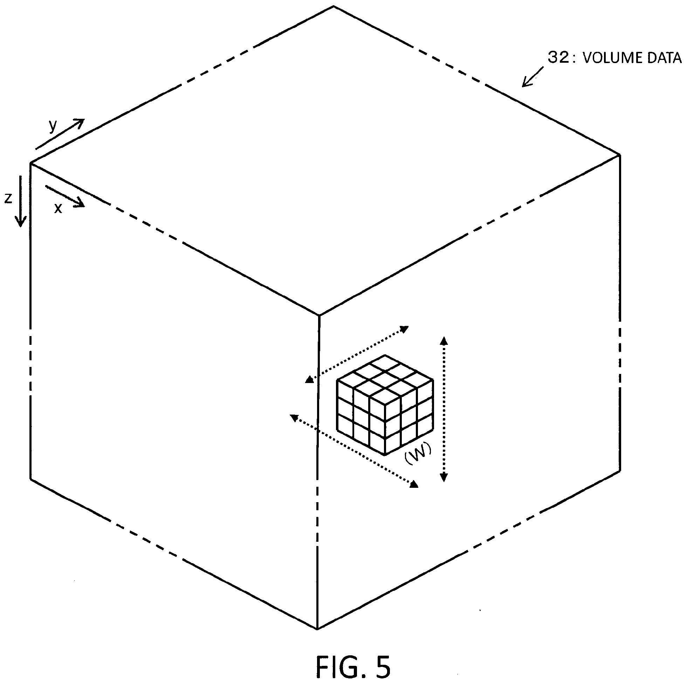

FIG. 5 is a diagram for illustrating the principle of a region growing method. FIG. 5 shows the volume data 32 in the xyz rectangular coordinate system corresponding to the three-dimensional space including the fetus.

The principle of the region growing method is as follows. First, a first target voxel is specified (step 1). Next, among a plurality of voxels adjacent to or close to the circumference of that target voxel, a suitable voxel that meets region conditions is searched (step 2), and the suitable voxel searched in step 2 is set as a new target voxel (step 3). Step 2 and step 3 are then repeated, and when no new suitable voxel that meets the region conditions remains, one region (voxel group) is determined (step 4). That is, one voxel group composed of a mass of voxels that meet the region conditions is determined.

When the principle of the region growing method is applied to a specific example shown in FIG. 5, first, the voxel group identification unit 50 sets a voxel corresponding to a reference point set by the reference point setting unit 40 as a first target voxel (step 1). If the reference point is set at a voxel corresponding to the fetus, one voxel corresponding to the fetus becomes the first target voxel.

Next, the voxel group identification unit 50 uses a window W having the target voxel at the center and searches a suitable voxel (step 2). For example, as shown in FIG. 5, a window W corresponding to three voxels in each of the x axis direction, the y axis direction, and the z axis direction; i.e., 27 voxels in total, is used, and the window W is positioned such that the target voxel is at the center of the window W. Among 26 surrounding voxels adjacent to the target voxel in the window W, a suitable voxel that meets the region conditions is then searched.

The region conditions include, for example, being a voxel corresponding to the same tissue as the target voxel. Specifically, for example, a voxel value (luminance value) of the target voxel is used as a reference value, and, among 26 surrounding voxels, one or more voxels having voxel values within a range of ± 10% of the reference value are regarded as suitable voxels. Suitable voxels may, of course, be determined according to other specific examples.

The voxel group identification unit 50 then uses each of the suitable voxels as a new target voxel (step 3), and moves the window W such that the new target voxel is at the center to search a suitable voxel (step 2). In this way, step 2 and step 3 are repeated, and when no new suitable voxel that meets the region conditions remains, one region (voxel group) is determined (step 4). If the reference point is set at a voxel corresponding to the fetus, a voxel group corresponding to the fetus is determined.

In addition, if the reference point setting unit 40 sets reference points corresponding to a plurality of objects, the voxel group identification unit 50 identifies, for each object, a voxel group corresponding to that object.

The voxel group identification unit 50 may cause the window W to move in a wide area in the volume data 32; for example, so as to cover all the area of the volume data 32 to determine voxel groups other than the fetus. For example, among a plurality of voxels having voxel values greater than (equal to or greater than) the threshold in the volume data 32, a voxel that differs from the voxel group of the fetus is set as a first target voxel, and the above-described step 1 to step 4 are performed.

For example, by regarding all voxels having voxel values greater than the threshold (equal to or greater than the threshold) in the volume data 32 as objects to be processed by the region growing method, it then becomes possible to determine all voxel groups in the volume data 32. If the number of suitable voxels that meet the region conditions is small (equal to or less than the reference number), the voxels may be excluded from the voxel groups.

Further, an upper limit of the number of voxels constituting a voxel group may be set. For example, by setting a reference point at the face of the fetus and regarding a plurality of voxels including a voxel of that reference point up to the limit number as one voxel group, it is possible to obtain a voxel group where only the face of the fetus is present (the face is dominant). Furthermore, in place of the upper limit number or in addition to the upper limit number, the size of a voxel group may be limited according to the distance from the reference point (first target voxel). The upper limit number of voxels and the distance from the reference point may be adjustable as appropriate by the user.

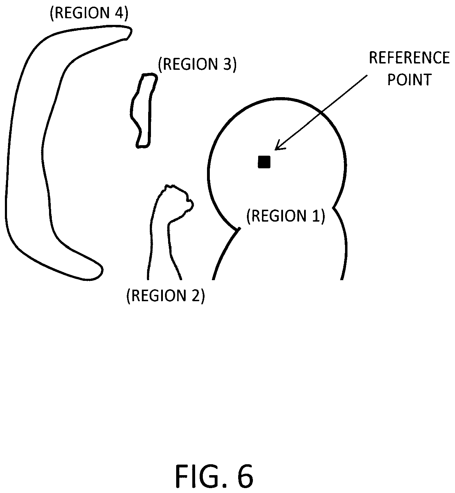

FIG. 6 is a diagram showing a specific example of a plurality of voxel groups. FIG. 6 shows a specific example of a plurality of voxel groups determined in the volume data 32 (FIG. 2, FIG. 5) corresponding to the three-dimensional space including the fetus.

In FIG. 6, a region 1 is a voxel group corresponding to the face of the fetus at which a reference point is set. A region 2 is a voxel group corresponding to the hand of the same fetus. If, in the volume data 32, a voxel portion of the face of the fetus and a voxel portion of the hand of the fetus are separate, those two voxel portions are recognized as different voxel groups, as shown in FIG. 6. Even if, in the volume data 32, the voxel portion of the face of the fetus is connected to the voxel portion of the hand, the above-described limits, such as the upper limit number of voxels and the distance from the reference point, enable separation of the face voxel portion from the hand voxel portion.

Further, in FIG. 6, a region 3 is a voxel group corresponding to a floating matter in the amniotic fluid, and a region 4 is a voxel group corresponding to the placenta or the uterine wall. If there are a plurality of floating matters in the volume data 32, a plurality of voxel groups corresponding to the plurality of floating matters can be obtained.

In this way, the voxel group identification unit 50 identifies a voxel group corresponding to the face of the fetus at which the reference point is set in the volume data 32 using, for example, the region growing method, and more preferably, identifies all the voxel groups that meet the conditions as voxel groups in the volume data 32.

The voxel group identification unit 50 may use a principle that differs from the region growing method such as, for example, known labeling processing, to identify one or more voxel groups in the volume data 32. For example, the plurality of regions 1 to 4 may be identified as shown in FIG. 6 by performing labeling processing on a plurality of voxels having voxel values greater than the threshold (equal to or greater than the threshold) in the volume data 32 (FIG. 2, FIG. 5).

The voxel group identification unit 50 may also perform expansion processing on each of the identified voxel groups. Because, for example, each voxel group identified by the region growing method or the labeling processing may have a voxel hole (voxel that does not meet the connection conditions) therein, it is preferable to expand voxels belonging to each voxel group by several voxels to fill the voxel hole (to include a voxel that does not meet the connection conditions in the voxel group). Furthermore, it is also possible to expand an outer edge of each voxel group by several voxels by expansion processing and obtain a voxel group that reliably includes an outer edge of an object such as, for example, the face of the fetus.

Once the voxel group identification unit 50 identifies one or more voxel groups, for example, only a voxel group including the reference point is set as a display target. For example, if the reference point is set at a voxel corresponding to the fetus, only a voxel group corresponding to the fetus is set as a display target.

A display target determination unit 60 may also determine, per voxel group, whether that voxel group is a display target. The display target determination unit 60 determines one or more voxel groups that become display targets based on instructions from the user, for example. A reference data storage unit 70 then stores reference data indicating, per voxel group, whether that voxel group is a display target.

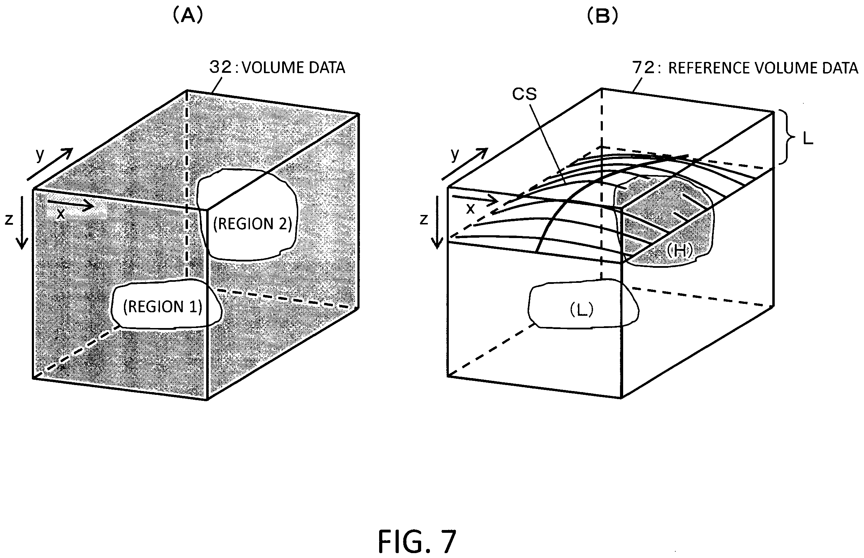

FIG. 7 is a diagram showing a specific example of reference data. FIG. 7 shows the volume data 32 stored in the three-dimensional data storage unit 30 and reference volume data 72 which is a specific example of reference data corresponding to that volume data 32 and is stored in the reference data storage unit 70.

The reference volume data 72 is composed of mark data that indicates, for each voxel of a plurality of voxels constituting the volume data 32, whether or not that voxel is a display target. That is, while the volume data 32 is composed of voxel data (voxel values) of a plurality of voxels provided with addresses in three dimensions in the xyz rectangular coordinate system, the reference volume data 72 is composed of, for example, mark data of a plurality of voxels provided with the same addresses as the volume data 32. Mark data of each voxel is, for example, set at "H" if that voxel is a display target, while it is set at "L" if that voxel is not a display target (if it is a non-display target).

In the specific example shown in FIG. 7, a voxel group of a region 1 and a voxel group of a region 2 are identified in the volume data 32. In the reference volume data 72, the mark data of a plurality of voxels corresponding to the voxel group of the region 1 is set at "L"; that is, it is set as a non-display target, while the mark data of a plurality of voxels corresponding to voxel group of the region 2 is set at "H"; that is, it is set as a display target.

Mark data of a plurality of voxels that are not included in the voxel groups in the reference volume data 72 may all be set at either "L" (non-display target) or "H" (display target). For example, in the case of the volume data 32 in the womb, a plurality of voxels corresponding to the amniotic fluid which is not included in the voxel groups have small luminance values (voxel data), and they are unlikely to disturb an image of the fetus, which is a display target, and, therefore, the mark data of the voxels corresponding to the amniotic fluid may all be set at "H" (display target).

Further, in the case of the volume data 32 in the womb, mark data of a plurality of voxels corresponding to the placenta in the reference volume data 72 is preferably set at "L" (non-display target). In the specific example shown in FIG.7, a region corresponding to the placenta (region on the placenta side) is identified by a clipping surface (CS) in the reference volume data 72.

The clipping surface CS can be identified by a known technique described in, for example, Patent Document 4 (JP 2011-83439 A). With the technology described in Patent Document 4, it is possible to set a clipping surface CS appropriately in the direction of a gap between the fetus and the placenta. For example, with the technology of Patent Document 4, it is possible to set a clipping surface CS as desired by the user in accordance with instructions from the user. As a matter of course, the device may automatically set the clipping surface CS by identifying the voxel portion of the amniotic fluid between the fetus and the placenta according to a technique different from the technology of Patent Document 4; for example, based on voxel data of the plurality of voxels constituting the volume data 32.

In addition, when the clipping surface CS is set, the reference point setting unit 40 may search voxels of the fetus from the clipping surface CS to the fetus side in the volume data 32 (reference volume data 72) to automatically set a reference point.

Once one or more voxel groups that become display targets are determined, the image forming unit 80 forms an ultrasound image that clearly displays image portions corresponding to the one or more voxel groups that are set as the display targets. Of the volume data 32 stored in the three-dimensional data storage unit 30, based on voxel data of a plurality of voxels corresponding to the voxel groups that are set as display targets in the reference volume data 72, the image forming unit 80 forms a three-dimensional ultrasound image stereoscopically showing the display targets.

For example, by performing rendering processing only on voxel data of a plurality of voxels corresponding to the voxel groups set as the display targets, a rendering image stereoscopically showing only image portions of voxel groups set as the display targets is formed.

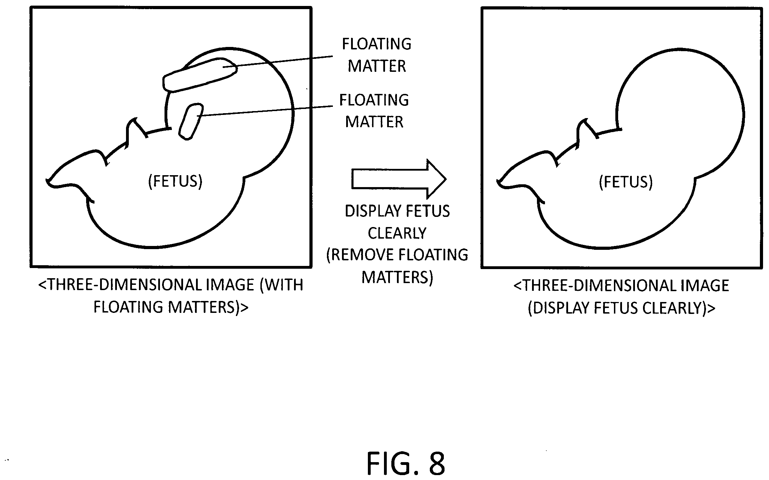

FIG. 8 is a diagram showing a specific example of three-dimensional images. FIG. 8 shows a specific example of three-dimensional images (rendering images) obtained based on the volume data 32 (FIG. 2) including the fetus and the floating matters.

In FIG. 8, a three-dimensional image including the floating matters (with the floating matters) is, for example, a three-dimensional image obtained based on volume data of all the voxels in the volume data 32 (voxels corresponding to the placenta may be removed). In this three-dimensional image, a portion of the face of the fetus is disturbed by the floating matters that are nearer to the viewpoint side than the fetus.

In contrast to this, in FIG. 8, a three-dimensional image clearly displaying the fetus is a three-dimensional image which is obtained based on voxel data of a plurality of voxels corresponding to the fetus in the volume data 32 and in which only a voxel group corresponding to the fetus in the reference volume data 72 is set as the display target. In the three-dimensional image clearly displaying the fetus, the portion of the face of the fetus is clearly displayed without being disturbed by the floating matters.

The image portion other than the display target (non-display target) may also be displayed so as to be transparent to clearly display the image portion of the display target. For example, in the specific example of FIG. 8, display processing for making the image portions of the floating matters transparent may be performed in order to prevent the image portions of the floating matters from being obstacles for the image portion of the fetus.

In this way, with the ultrasound diagnostic device in FIG. 1, it is possible, for example, to form a three-dimensional image such that the image portion of the display target (the fetus or the like) is not disturbed by the other image portions (the floating matter or the like). Some voxel groups may be set as display targets, or conversely, some voxel groups may be set as non-display targets (not display targets).

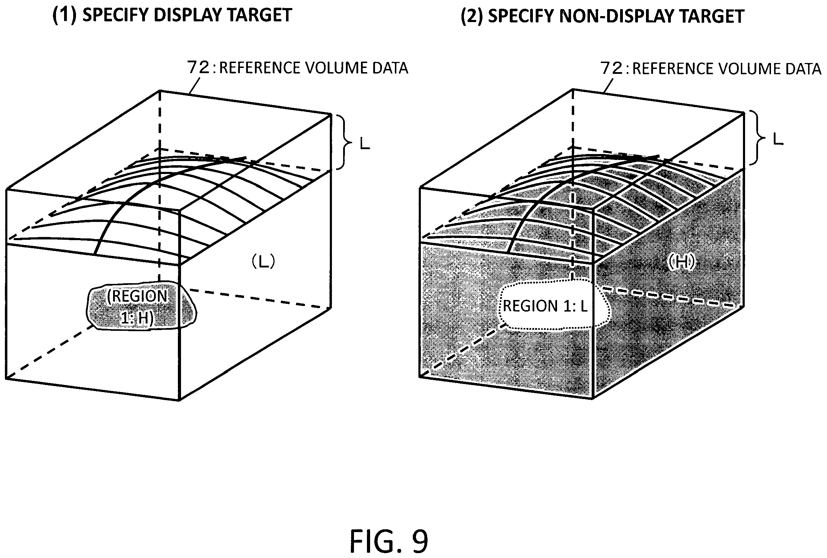

FIG. 9 is a diagram showing a specification example 1 of a display target and non-display targets. FIG. 9(1) shows a specific example where a display target is specified. In FIG. 9(1), a voxel group of the region 1 identified in the volume data 72 is set as a display target, and mark data of a plurality of voxels belonging to the voxel group of the region 1 is set at "H," while mark data of the plurality of voxels other than it is set at "L" (non-display target).

Meanwhile, FIG. 9(2) shows a specific example where non-display target are specified. In FIG. 9(2), the voxel group of the region 1 identified in the volume data 72 is set as a non-display target, and mark data of the plurality of voxels belonging to the voxel group of the region 1 is set at "L." In the case of the reference volume data 72 related to the volume data 32 in the womb, mark data of a plurality of voxels corresponding to a region on the placenta side is preferably set at "L" (non-display target). For example, as shown in the specific example in FIG. 7, a region corresponding to the placenta (region on the placenta side) is identified by the clipping surface CS in the reference volume data 72. In the specific example in FIG. 9(2), the region 1 and the region on the placenta side are set as non-display targets, and mark data of a plurality of voxels in regions other than them is set at "H" (display target).

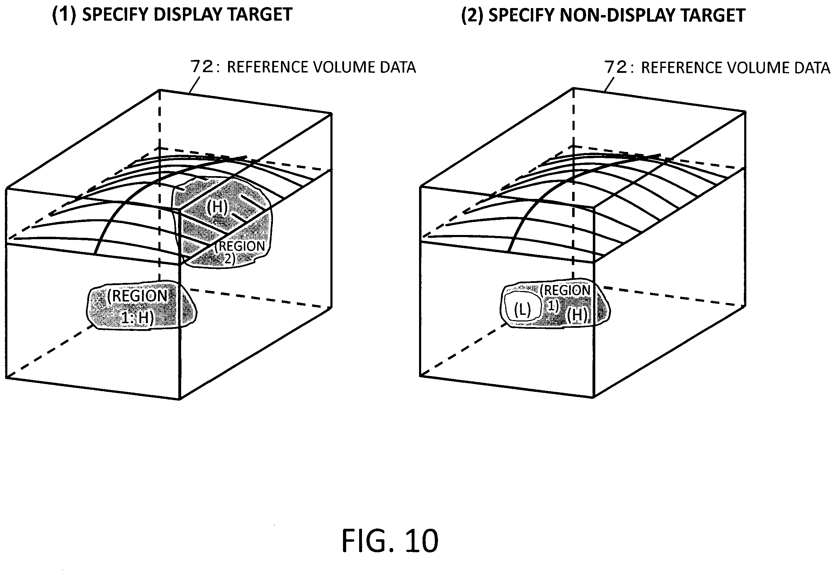

FIG. 10 is a diagram showing a specification example 2 of display targets and a non-display target. FIG. 10 shows a specific example where a display target and a non-display target are additionally specified in the status in FIG. 9(1).

FIG. 10(1) shows a specific example where a display target is added. FIG. 10(1) shows a specific example where, in the volume data 72 in which the voxel group of the region 1 is set as the display target, a voxel group of a region 2 is further set as a display target. In the volume data 72 in FIG. 10(1), mark data of the plurality of voxels belonging to the voxel groups of the region 1 and the region 2 are set at "H," while mark data of the plurality of voxels other than them is set at "L" (non-display target).

If a portion of the region 2 penetrates through the clipping surface CS (FIG. 7) and protrudes into the region of the placenta side, preferably, that protruding portion is also set as a display target. In doing so, even if, for example, a portion of the face or the hand of the fetus protrudes from the clipping surface CS, that protruding portion can be changed to a display target.

FIG. 10(2) shows a specific example where a non-display target is added. FIG. 10(2) shows a specific example where, in the volume data 72 in which the voxel group of the region 1 is set as the display target, a part of the region 1 is set as a non-display target.

For example, if the voxel group of the region 1 including the face and the arm of the fetus is identified, by limiting the size of the voxel group using the upper limit number of voxels or the distance from the reference point set at the face of the fetus, it is possible to obtain a voxel group in which only the face of the fetus is present (the face is dominant) in the region 1 (refer to the description related to FIG. 5). Subsequently, by setting voxels other than the voxel group corresponding to the face of the fetus as non-display targets, it is possible to display the face of the fetus without being disturbed by the arm of the fetus. Further, even if the fetus is connected to the placenta or the uterine wall to form the region 1, it is also possible to set the placenta or the uterine wall as a non-display target while retaining the face of the fetus.

As described using FIG. 10, once the voxel group, which is a display target or a non-display target, is additionally specified in the reference volume data 72, mark data constituting the reference volume data 72 is updated according to that specification each time specification is made. It is therefore preferable to store update history related to the reference volume data 72 in the reference data storage unit 70. For example, for each voxel of the plurality of voxels constituting the volume data 72, history data related to mark data of that voxel is stored. That is, a change history between "H (display target)" and "L (non-display target)" is stored for each voxel. In doing so, if, for example, the user wants to return a specification of a display target and a non-display target to their original statuses, it becomes possible to reproduce the past reference volume data 72 by tracing the change history of the mark data of the voxels, in response to an operation (undo operation) by the user. Further, for example, based on the past reference volume data 72, reference volume data 72 formed after that may be reproduced in response to the operation by the user.

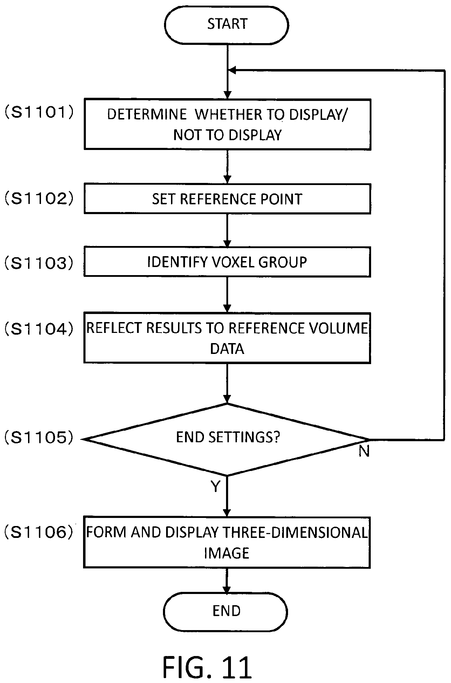

FIG. 11 is a diagram (flowchart) showing a specific example of processing performed by the ultrasound diagnostic device in FIG. 1. First, whether a voxel group now to be identified is a display target or a non-display target is set (S1101). For example, whether it is a display target or a non-display target is determined according to a specification that is input from the user via the operating device 92.

Next, a reference point is set based on instructions from the user (S1102: see FIG. 3 and FIG. 4). For example, if the user wishes to identify the face of the fetus as a display target, the user sets a reference point in an image portion corresponding to the face of the fetus in a tomographic image or a three-dimensional image. In addition, for example, if the user wishes to identify a floating matter as a non-display target, the user sets a reference point in an image portion corresponding to the floating matter in the tomographic image or the three-dimensional image.

Once the reference point is set, one voxel group composed of a plurality of voxels having voxel data that satisfy the connection conditions using a voxel of the reference point as an origin is identified (S1103: see FIG. 5 and FIG. 6).

Next, one identified voxel group is reflected in the reference volume data 72 (S1104: see FIG. 7, FIG. 9, and FIG. 10). For example, if the voxel group is set as a display target at S1101, the reference volume data 72 is updated such that the voxel group identified at S1103 becomes a display target. On the other hand, if the voxel group is set as a non-display target at S1101, the reference volume data 72 is updated such that the voxel group identified at S1103 becomes a non-display target.

Whether or not the settings of the voxel group are ended is then confirmed (S1105). If, for example, the user wants to perform settings of a plurality of voxel groups, the processing from S1101 to S1104 is performed for each voxel group, and the processing from S1101 to S1104 is repeated, thereby performing the settings of the plurality of voxel groups.

In this way, when the settings of the voxel groups are ended (S1105), a three-dimensional image clearly displaying image portions corresponding to one or more voxel groups that are display targets is formed (S1106: see FIG. 8). For example, if the face of the fetus is set as a display target and the floating matters or the like are set as non-display targets, a rendering image clearly displaying the face of the fetus is formed.

As such, with the ultrasound diagnostic device in FIG. 1, it is possible to form a three-dimensional image in which, for example, only the face of the fetus is present (or the face of the fetus is dominant) in the volume data 32 of the three-dimensional space including, for example, the fetus. However, if, for example, only the face of the fetus is clearly displayed in the three-dimensional image, and other tissues are not displayed at all or are hardly displayed, the image becomes an unnatural and strange image in which only the face of the fetus is shown. Accordingly, the ultrasound diagnostic device in FIG. 1 may perform image processing for adding a background to the display target, in order to eliminate or alleviate that strangeness and unnaturalness.

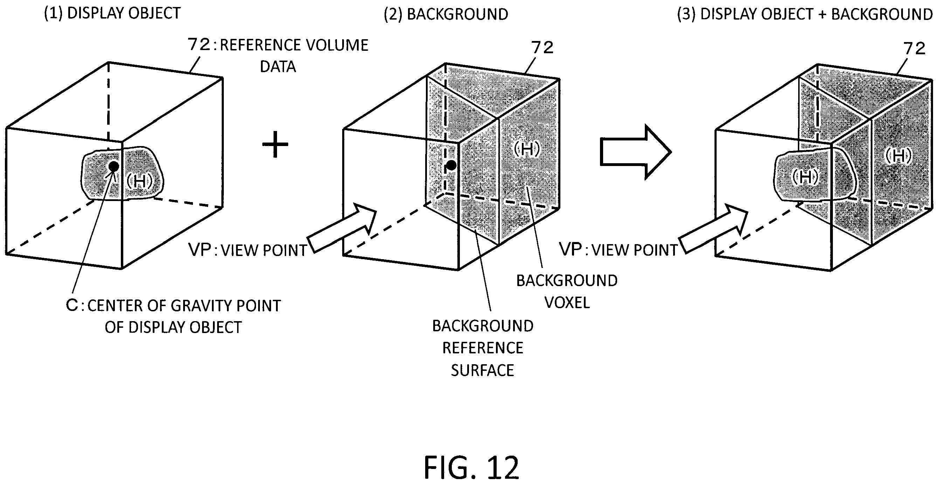

FIG. 12 is a diagram showing an image processing example in which a background is added to a display target. In a reference volume data 72 shown in FIG. 12(1), only one voxel group is set as a display target (display target voxel group). For example, a voxel group corresponding to the face of the fetus is set as a display target.

Next, a representative point of the display target voxel group which is, for example, the center of gravity point of the display target voxel group is derived, and, when viewed from the viewpoint VP side of the three-dimensional image, a plurality of voxels located further than the center of gravity point (representative point) are set as a background voxel group. For example, as shown in FIG. 12(2), a background reference surface is set to include the center of gravity point (representative point) of the display target voxel group and to become orthogonal to a line of vision, and all the voxels located further than the background reference surface when viewed from the viewpoint VP side are set as a background voxel group.

As shown in FIG. 12(3), a three-dimensional image (rendering image) viewed from the viewpoint VP is then formed based on voxel data of a plurality of voxels of the voxel group corresponding to the display target (display target voxel group) and the background voxel group.

When viewed from the viewpoint VP, the background voxels are located further than the display target voxels, and, therefore, an image portion of the display target voxels is not disturbed by an image portion of the background voxels in the three-dimensional image. In addition, because the image portion of the background voxels is also displayed, as compared to, for example, the case where only the image portion of the display target voxels is displayed and the case where only the face of the fetus is displayed, it becomes possible to obtain a natural three-dimensional image with a background. For example, in addition to the face of the fetus, the uterine wall and the floating matters on the background of that face are displayed, and it becomes possible to form a natural image without strangeness while clearly displaying the face of the fetus.

The position, inclination, and shape of the background reference surface may be adjustable, for example, by the user. For example, it is possible to display on the display unit 82a three-dimensional image obtained based on the display target voxels and the background voxels, and adjust the position, inclination, and shape of the background reference surface by the user operating the operating device 92 while visually confirming the three-dimensional image displayed on the display unit 82.

Although the preferred embodiment of the present invention has been described, of the structure shown in FIG. 1 (units with the reference numbers), the transmission and reception unit 12, the beam data processing unit 20, the reference point setting unit 40, the voxel group identification unit 50, the display target determination unit 60, and the image forming unit 80 can each be implemented with hardware such as, for example, an electronic circuit and processor, and devices, such as a memory, may also be used when necessary in implementation of them. Further, at least some of the functions corresponding to the above units may be implemented with a computer. That is, at least some of the functions corresponding to the above units may be implemented by cooperation between hardware, such as a CPU, processor, and memory, and software (program) that defines operations of the CPU and the processor.

The three-dimensional data storage unit 30 and the reference data storage unit 70 can each be implemented with a storage device such as, for example, a semiconductor device and a hard disk drive. As a matter of course, the three-dimensional data storage unit 30 and the reference data storage unit 70 may be integrated in one storage device. A preferable specific example of the display device 82 is, for example, a liquid crystal display, and the operating device 92 can be implemented with at least one of a mouse, a keyboard, a trackball, a touch panel, and other switches.

The control unit 90 controls the entire ultrasound diagnostic device in FIG. 1. Instructions received from the user via the operating device 92 are reflected in the overall control by the control unit 90. The control unit 90 can be implemented by cooperation between hardware, such as a CPU, processor, and memory, and software (program) that defines operations of the CPU and the processor.

The preferred embodiment of the present invention described above is merely illustrative in all respects and does not limit the scope of the present invention. The present invention includes various variants without departing from the spirit and scope of the present invention.

### REFERENCE SIGNS LIST

10 probe, 12 transmission and reception unit, 20 beam data processing unit, 30 three-dimensional data storage unit, 40 reference point setting unit, 50 voxel group identification unit, 60 display target determination unit, 70 reference data storage unit, 80 image forming unit, 90 control unit, 82 operating device.

## Claims

1. An ultrasound diagnostic device comprising:
an identification unit that identifies, in ultrasonic volume data, one or more voxel groups composed of a plurality of voxels having voxel data that satisfy connection conditions, based on voxel data of a plurality of voxels constituting the volume data; and
an image forming unit that forms, based on voxel data of a plurality of voxels corresponding to a voxel group that is a display target among the identified one or more voxel groups, an ultrasound image clearly displaying the display target in a selective manner.

2. The ultrasound diagnostic device according to Claim 1, further comprising:
a determination unit that determines, for each voxel group identified by the identification unit, whether that voxel group is a display target; and
a storage unit that stores reference data indicating, for each voxel group, whether that voxel group is a display target.

3. The ultrasound diagnostic device according to Claim 2, wherein
the storage unit stores, as the reference data, reference volume data composed of mark data indicating, for each voxel of the plurality of voxels constituting the volume data, whether that voxel is a display target, and
the determination unit updates the reference volume data such that the mark data of a plurality of voxels belonging to a voxel group that is determined to be the display target shows the display target.

4. The ultrasound diagnostic device according to any one of Claims 1 to 3, further comprising
a setting unit that sets a reference point within the volume data based on instructions from the user, wherein
the identification unit identifies the voxel group composed of the plurality of voxels that satisfy the connection conditions, using a voxel corresponding to the reference point as an origin.

5. The ultrasound diagnostic device according to Claim 4, wherein
the setting unit sets, as the reference point, a specified point which is set by the user in a tomographic image corresponding to a cross section in the volume data.

6. The ultrasound diagnostic device according to Claim 4, wherein
a three-dimensional image showing the inside of the volume data stereoscopically is formed based on pixel data obtained from a plurality of rays passing through the inside of the volume data, and
the setting unit sets the reference point on a ray corresponding to pixel data of a specified point set by the user in the three-dimensional image.

7. The ultrasound diagnostic device according to Claim 6, wherein
the setting unit searches a voxel region corresponding to the face of a fetus based on voxel data of a plurality of voxels constituting the ray corresponding to the specified point, and sets the reference point within the voxel region.

8. The ultrasound diagnostic device according to any one of Claims 4 to 7, wherein
the identification unit identifies, as the voxel group, a plurality of voxels that satisfy the connection conditions using the voxel corresponding to the reference point as the origin and that are located within a specified distance from the reference point.

9. The ultrasound diagnostic device according to any one of Claims 4 to 7, wherein
the identification unit identifies, as the voxel group, a plurality of voxels that satisfy the connection conditions using the voxel corresponding to the reference point as the origin and that are less than a specified number of voxels.

10. The ultrasound diagnostic device according to any one of Claims 1 to 9, wherein
the one or more voxel groups identified by the identification unit include a voxel group corresponding to the face of the fetus which is a display target, and
the image forming unit forms an ultrasound image clearly displaying the face of the fetus in a selective manner based on voxel data of a plurality of voxels constituting the voxel group corresponding to the face of the fetus.

11. The ultrasound diagnostic device according to Claim 10, wherein
based on the voxel data of the plurality of voxels corresponding to the face of the fetus and voxel data of a plurality of voxels that satisfy conditions for a background tissue in the volume data, the image forming unit forms an ultrasound image that shows the face of the fetus and the background tissue.
